Europäisches Patentamt

European Patent Office

Office européen des brevets

(11) Veröffentlichungsnummer: **0 331 127 B1**

# EUROPÄISCHE PATENTSCHRIFT

(45) Veröffentlichungstag der Patentschrift: **16.06.93**

(21) Anmeldenummer: **89103528.9**

(22) Anmeldetag: **28.02.89**

(51) Int. Cl.5: **G01N 33/547**, G01N 33/544, //G01N33/531

(54) Verfahren zur Herstellung einer Festphasenmatrix.

(30) Priorität: **29.02.88 DE 3806431**

(43) Veröffentlichungstag der Anmeldung:
**06.09.89 Patentblatt 89/36**

(45) Bekanntmachung des Hinweises auf die Patenterteilung:
**16.06.93 Patentblatt 93/24**

(84) Benannte Vertragsstaaten:
**AT BE CH DE ES FR GB GR IT LI LU NL SE**

(56) Entgegenhaltungen:
EP-A- 0 016 552      EP-A- 0 122 209
EP-A- 0 185 372      EP-A- 0 245 926
EP-A- 0 269 092      WO-A-87/04794
GB-A- 2 103 791      US-A- 4 282 287

RÖMPP's CHEMIE-LEXIKON, 8. Aufl., 1987;
Seite 3301*

(73) Patentinhaber: **BOEHRINGER MANNHEIM GMBH**
**Sandhofer Strasse 112-132**
**W-6800 Mannheim-Waldhof(DE)**

(72) Erfinder: **Berger, Michael, Dr.**
**Knappenstr. 16**
**W-8122 Penzberg(DE)**
Erfinder: **Deger, Arno, Dr.**
**Foehrenstr. 3**
**W-8124 Seeshaupt(DE)**
Erfinder: **Maier, Josef**
**Schmädlstr. 15**
**W-8120 Weilheim(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J. Prechtel Kopernikusstrasse 9 Postfach 86 08 20**
**W-8000 München 86 (DE)**

**Beschreibung**

Die Erfindung betrifft ein Verfahren zur Herstellung einer an ein unlösliches Trägermaterial gebundenen, spezifisch bindefähigen Substanz, insbesondere für die Verwendung in einem heterogenen Analysenverfahren nach dem Prinzip des Immunoassays.

Zur Bestimmung einer spezifisch bindefähigen Substanz bedient man sich häufig Verfahren nach dem Prinzip des Immunoassay. Dabei wird einer der Partner eines spezifisch miteinander bindefähigen Substanzpaares mit dem für ihn spezifischen Rezeptor, der in an sich bekannter Weise markiert ist, umgesetzt. Das Konjugat aus diesen beiden Substanzen kann dann noch mit einem Rezeptor, der für das Konjugat oder eines der beiden Teile des Konjugats spezifisch ist, umgesetzt werden. Für diese immunologischen Verfahren gibt es viele Variationen. Vorteilhaft ist es dabei, wenn einer der Rezeptoren an eine Festphase gebunden vorliegt. Dies erleichtert die Trennung von gebunden und nicht gebunden vorliegenden Reaktionspartnern. Zur Bestimmung der spezifisch bindefähigen Substanz wird dann die Menge von an der Festphase gebundenem markiertem Reaktionspartner oder von in der Lösung vorliegendem markiertem Reaktionspartner gemessen und zu der Menge an zu bestimmendem Reaktionspartner in an sich bekannter Weise in Beziehung gesetzt.

Als Festphase werden bei den immunologischen Verfahren üblicherweise Kunststoffröhrchen oder Mikrotiterplatten, an deren Innenoberfläche der Reaktionspartner fixiert ist, oder Kugeln, an deren Außenoberfläche der Reaktionspartner fixiert ist, verwendet. Diese Kunststoffröhrchen, Mikrotiterplatten oder Kugeln bestehen üblicherweise aus relativ inertem Kunststoffmaterial, so daß die Bindung des Reaktionspartners Schwierigkeiten bereitet.

Darüberhinaus muß die Bindung des spezifischen Reaktionspartners an die jeweilige Oberfläche so erfolgen, daß er nicht die Fähigkeit zur spezifischen Bindung der für ihn spezifisch bindefähigen Substanz verliert. Aus diesem Grund erfolgt die Bindung des Reaktionspartners an die Festphase meistens adsorptiv. Es wurde daher schon vorgeschlagen, die Fixierung des Reaktionspartners an die Festphase über ein Kupplungsmittel, das die Bindung vermittelt, zu bewirken. Dabei muß auch wieder darauf geachtet werden, daß die Bindung des Reaktionspartners an das Kupplungsmittel nicht die spezifisch reagierende Region des Moleküls zerstört bzw. daß der Reaktionspartner so gebunden wird, daß seine reaktive Stelle von der Festphase weg dem Bindungspartner zugewandt ist. Weiterhin wird in DE-OS 25 33 701 vorgeschlagen, um eine bessere Bindung zu erzielen, die einzelnen immunologisch wirksamen Proteine zu vernetzen und dann an Polystyrolkugeln zu adsorbieren. Als weitere Möglichkeit ist in dieser Literaturstelle angegeben, gleichzeitig mit dem Protein mit immunologischen Eigenschaften ein inertes Protein zu vernetzen, so daß ein vernetztes Produkt aus inertem und aktivem Protein entsteht, das dann wiederum an Polystyrolkügelchen adsorbiert wird. Diese Art der Vernetzung führt jedoch in Abhängigkeit von den gewählten Reaktionsbedingungen zu unterschiedlichen, nicht reproduzierbaren Vernetzungen mit schwankenden Anteilen an nicht vernetztem sowie unlöslich gewordenem Protein. Durch den unterschiedlichen Vernetzungsgrad entstehen zudem Produkte mit unterschiedlichen Bindungseigenschaften. Ein ähnliches Verfahren ist in der EP-A-122 209 beschrieben und weist auch die gleichen Nachteile auf. All diese bekannten Verfahren befriedigen somit noch nicht, führen noch zu keiner optimalen Haftung der spezifisch bindefähigen Substanz und sind für die reproduzierbare Herstellung von beschichteten Festphasen wenig geeignet.

Ein weiteres Problem besteht außerdem darin, daß der an der Festphase gebundene Antikörper für verschiedene Tests in der Regel verschieden sein muß. Es muß daher für jeden Test eine spezifisch beschichtete Festphase zur Verfügung gestellt werden. Dies ist sehr aufwendig. Da außerdem nicht gewährleistet ist, daß jeder in bekannter Weise immobilisierte Antikörper bindefähig bleibt, da oft auch unspezifische Bindungen erfolgen, und da außerdem die Ablösungsrate bei den bisher bekannten Verfahren sehr hoch ist, muß der Antikörper in hohem Überschuß eingesetzt werden. Trotzdem ist die Zahl der Bindeplätze begrenzt. Damit ergibt sich eine erhöhte Störanfälligkeit. Dies ist nachteilig, da Antikörper schwierig und aufwendig herzustellen sind.

Aus WO-A-8 704 794 ist eine Festphasenmatrix bekannt, die aus einem inerten partikelförmigen polymeren Träger besteht, an den über Biotin eine vernetzte Avidinschicht adsorbiert ist. Diese Anordnung kann ein Ausbluten nicht verhindern.

Aus EP-A-0 245 926 ist ein Verfahren zur Bestimmung von Antigenen, Antikörper oder Haptenen bekannt, bei welchem in flüssiger Phase ein löslicher Komplex der gesuchten Substanz mit einem damit bindefähigen Partner und einer löslichen Matrix gebildet wird, und anschließend der Komplex auf einem festen Träger unlöslich gemacht wird durch Bindung eines an diese Matrix gebundenen Liganden an einem anti-Liganden und dann der unlöslich gemachte Komplex über eine Markierung in üblicher Weise bestimmt wird. Dieses Verfahren ist nicht gegen eine Ablösung des löslichen Komplexes vom unlöslichen Träger gesichert.

Es war daher Aufgabe der Erfindung, ein Verfahren zu schaffen, das die Haftung der spezifisch bindefähigen Substanz an der Festphase reproduzierbar verbessert und darüberhinaus ein Verfahren zu schaffen, mit dem eine Festphasenmatrix hergestellt werden kann, die universell für alle Immunoassays einsetzbar ist. Da bei vielen immunologischen Verfahren unter Detergentienzusatz gearbeitet wird, um Trübungen und unspezifische Bindungen zu vermeiden, war es außerdem Ziel der Erfindung, die Haftung so weit zu verbessern, daß auch bei Anwesenheit von Detergentien sich die gebundene, spezifisch bindefähige Substanz nicht ablöst.

Dieses Ziel wird erreicht durch ein Verfahren zur Herstellung einer an ein unlösliches Trägermaterial gebundenen, spezifisch bindefähigen Substanz, insbesondere für die Verwendung in einem heterogenen Analyseverfahren nach dem Prinzip des Immunoassays, welches dadurch gekennzeichnet ist, daß man ein erstes Polymerisat I, das aus einer Vielzahl von Partnern $P_1$ eines spezifischen Bindungspaares und einem wasserlöslichen biologischen Polymer oder Derivat davon mit einem Molekular gewicht von mehr als etwa 20 000 besteht, an ein unlösliches Trägermaterial bindet und mit einem zweiten Polymerisat II, das nur aus einer Vielzahl von Molekülen des anderen Partners $P_2$ des spezifischen Bindungspaares oder aus $P_2$, die mit den anderen Komponenten vernetzt sind, besteht, über die spezifi sche Bindung von $P_1$ mit $P_2$ vernetzt, wobei das Polymerisat II sowohl Bindungsstellen für $P_1$ als auch für einen immunolo gisch nachzuweisenden Komplex aufweist.

Mit diesem Verfahren kann eine Festphasenmatrix hergestellt werden, die für alle Arten von Immunoassays, z.B. Sandwich-Tests oder kompetitive Tests im Einschritt-oder Zweischrittverfahren, einsetzbar ist. Für den Sandwich-Test im Einschrittverfahren kann beispielsweise das Polymerisat II des Partners $P_2$ die Bindungsstelle liefern, an der dann der zu bestimmende Komplex immobilisiert wird. Während des Bestimmungsverfahrens wird eine Probe, die die zu bestimmende Substanz enthält, mit einem markierten Rezeptor sowie einem unmarkierten Rezeptor, der mit dem Polymerisat II bindefähig ist, umgesetzt. Der sich bildende Komplex aus zu bestimmender Substanz, markiertem Rezeptor und Rezeptor mit Bindungsstelle für das Polymerisat II bindet dann aufgrund der spezifischen Bindefähigkeit an das Polymerisat II, so daß auf diese Weise der gesamte Komplex immobilisiert wird. Nach Trennung der Phasen kann dann in einer der beiden Phasen die Markierung gemessen werden.

Wenn der Sandwich-Test im Zweischrittverfahren durchgeführt wird, kann eine Festphasenmatrix verwendet werden, bei der ein unmarkierter Rezeptor in dem Polymerisat II gebunden ist. Es wird dann die Probe und der markierte Rezeptor in Gegenwart dieser Festphasenmatrix inkubiert. Nach Trennung der Phasen kann dann die an der Festphase gebundene Markierung gemessen werden.

Bei Durchführung eines kompetitiven Tests konkurrieren Probe und markiertes Probenanalogon um einen unmarkierten Rezeptor. Für die Testvariante des Einschrittverfahrens verwendet man in diesem Fall ein Polymerisat II, an das der unmarkierte Rezeptor gebunden ist. Für die andere Variante des Zweischrittverfahrens wird der unmarkierte Rezeptor während des Tests in Konkurrenz mit dem markierten Rezeptor gebunden, sodaß für diesen Fall dann ein Polymerisat II eingesetzt wird, das Bindungsstellen für diese Rezeptoren aufweist.

Zur Herstellung der erfindungsgemäßen Festphasenmatrix wird ein unlösliches Trägermaterial mit einem ersten Polymerisat I beschichtet, wobei die Bindung an das Trägermaterial nicht kovalent erfolgt, sondern durch Adsorption oder durch Wechselwirkung bewirkt wird, und mit einem zweiten Polymerisat II vernetzt. Für das Polymerisat I sind biologische Polymere geeignet, die wasserlöslich sind und ein Molekulargewicht von mehr als etwa 20.000 aufweisen. Bevorzugt werden für das Polymerisat I Proteine, Peptide, Nukleinsäurepolymere, Kohlenhydrate sowie Copolymerisate aus Aminosäuren und Kohlenhydraten verwendet. Geeignet sind auch derivatisierte Polymere wie Kohlenhydrate in derivatisierter Form, z.B. Aminodextran. Das Polymerisat I weist weiterhin eine Vielzahl von Partnern $P_1$ eines spezifischen Bindungs-paares auf. Die Partner $P_1$ können mit den Polymeren vernetzt oder an sie gebunden sein. Das Polymerisat I hat ein Molekulargewicht von mehr als 20.000, da bei einem geringeren Molekulargewicht die Bindung an das unlösliche Trägermaterial unter Umständen nicht mehr gewährleistet ist. Bevorzugt beträgt das Molekulargewicht mehr als 45.000 und besonders bevorzugt mehr als 200.000.

Die Bindung der Partner $P_1$ an das Polymere erfolgt in an sich bekannter Weise. Geeignete Kupplungsmethoden sind z.B. in Ishikawa, J. Immunoassay, 4, 209-327 (1983) beschrieben. Dabei werden entweder funktionelle Gruppen des Partners $P_1$, die für eine Bindung mit dem Polymer geeignet sind herangezogen, oder aber, wenn geeignete funktionelle Gruppen nicht vorhanden sind, werden diese in das $P_1$-Molekül eingeführt. So kann beispielsweise bei Verwendung von Biotin als $P_1$ das N-Hydroxysuccinimidylderivat durch Umsetzung mit im Polymer vorhandenen Aminogruppen gebunden werden. Andere geeignete Derivatisierungen sind dem Fachmann bekannt und brauchen hier nicht erläutert zu werden.

Das Verhältnis der Partner $P_1$ zu dem für das Polymerisat I verwendeten Polymer ist an sich unkritisch und kann in weiten Grenzen variiert werden. Es hat sich erwiesen, daß es vorteilhaft ist, pro Polymer 1 bis

200 Mol Partner $P_1$ einzusetzen. Dabei ist die $P_1$-Menge abhängig vom Einsatzzweck und vom verwendeten Polymer. Da das Polymerisat I insgesamt relativ hydrophob sein soll, um eine adsorptive Bindung bewirken zu können, sollte, wenn $P_1$ hydrophil ist, der Anteil an $P_1$ um so geringer sein, je kleiner das Molekulargewicht des verwendeten Polymers ist. Dies ist beispielsweise bei Biotin der Fall.

Geeignete Bindungspartner, die als $P_1$ und $P_2$ sowie in der Bindung zwischen dem Polymeren II und dem immunologisch nachzuweisenden Komplex verwendet werden können, sind z.B. Biotin-Avidin, Biotin-Streptavidin, Antigen-Antikörper, Hapten-Antikörper, Protein A-Immun-$\gamma$-Globulin und Protein G-Immun-$\gamma$-Globulin. Als Antigen bzw. Hapten werden auch Konjugate von Proteinen mit Antigen bzw. Hapten bzw. Fragmenten davon verstanden. Das Antigen kann auch selbst ein Antikörper, dessen Fab-, Fab'- oder (Fab')$_2$-Fragment sein. Unter Antikörper sind monoklonale und polyklonale, vollständige Antikörper und Antikörperfragmente zu verstehen. Wird als $P_1$ oder $P_2$ Protein A bzw. Protein G verwendet, so sollte in dem Immunoassay nur der eine Rezeptor, der an die Festphase gebunden werden soll, ein vollständiger Antikörper sein, während als markierter Rezeptor ein Fab- oder F(ab')$_2$-Fragment verwendet werden sollte, um keine unspezifische Bindung des markierten Rezeptors mit der Festphase zu verursachen, die zu einer Verfälschung des Ergebnisses führen würde.

Bevorzugt wird zur Herstellung des Polymerisats I ein Protein verwendet, das hydrophober als die Partner $P_1$ und $P_2$ ist. Geeignet sind insbesondere lösliche Proteine mit einem Molekulargewicht über etwa 200.000 bis etwa 20.000.000, die gegebenenfalls aus Proteinen mit einem Molekulargewicht von 10.000 bis 700.000 erhalten wurden und mit Partnern $P_1$ eines spezifisch bindenden Paares konjugiert sind.

Molekulargewicht und Hydrophobizität werden nach den dem Fachmann bekannten Methoden ermittelt. Zum Vergleich der Hydrophobizität zwischen löslichem Protein und spezifisch bindefähiger Substanz eignen sich beispielsweise folgende Methoden:
- der Fluoreszenzlöschung nach Bindung an Farbstoffe (Biochem. Biophys. Acta 624, (1980), 13-20),
- des Elutionsverhaltens bei der hydrophoben Chromatographie (Biochem. Biophys. Acta, 576 (1979), 269-279),
- der Oberflächenspannung (Biochem. Biophys. Acta, 670 (1981), 64-73),
- der Retentionszeiten bei Hydrophobic Interaction Chromatography (HIC) (Angew. Chemie 98 (1986) 530-548, J. Chromat. 296 (1984) 107-114, Anal. Biochem. 137, (1984) 464-472).

Ein Vergleich der Hydrophobizität von erfindungsgemäß geeigneten Substanzen findet sich in Sep. Sci. Technol. 14, 305-317 (1979). Danach steigt die Hydrophobizität z.B. in folgender Reihe an:

$\alpha$-$_2$-Macroglobulin (MG 820.000)

Rinderserumalbumin/Humanserumalbumin (MG 70.000)

Eialbumin

$\alpha_2$HS-Glycoprotein (MG 49.000)

$\beta_{1c}/\beta_{1A}$-Globulin

Immunglobulin (MG 150.000)

Transferrin (MG 90.000)

Wird also als spezifisch bindefähige Substanz ein Immunglobulin verwendet, sind beispielsweise für diese spezielle Ausführungsform Humanserumalbumin oder $\alpha_2$HS-Glycoprotein als lösliche Proteine ohne weitere Vorbehandlung nicht geeignet.

Beide Proteine müssen hier sowohl einer Hydrophobisierung als auch einer Erhöhung des Molekulargewichts unterworfen werden. Bei Transferrin genügt in diesem Fall eine Vernetzung, bei $\alpha_2$-Macroglobulin ist eine Hydrophobisierung ausreichend.

Proteine, die zur Kupplung mit Immunglobulin als spezifisch bindefähiger Substanz ohne Vorbehandlung geeignet sind, sind beispielsweise $\beta$-Lipoproteine (MG ca. 3,2 Mio) oder $\alpha_2$-Lipoprotein (MG ca. 5 bis 20 Mio).

Die Hydrophobisierung kann beispielsweise durch Anwendung von Hitze, Behandlung mit Säuren, denaturierenden Agentien und/oder chaotropen Ionen und/oder durch chemische Kupplung mit einer hydrophoben Verbindung erfolgen.

Die Erhöhung des Molekulargewichts kann beispielsweise durch Anwendung von Hitze, Behandlung mit Säuren, denaturierenden Agentien und/oder chaotropen Ionen und/oder durch Vernetzung mit einer bi- oder polyfunk3tionellen Verbindung erfolgen.

Ein Protein, das nicht genügend hydrophob ist oder dessen Molekulargewicht nicht ausreichend hoch ist, wird solange behandelt, bis ein Proteinpolymeres mit einem Molekulargewicht von 20.000, bevorzugt 45.000 und besonders bevorzugt mehr als 200.000 erhalten wird. Ganz besonders geeignet ist ein Proteinpolymeres mit einem Molekulargewicht von 500.000 bis 20 Mio..

Falls das Protein vernetzt werden soll, kann eine Hydrophobisierung vor, während oder nach der Vernetzung stattfinden. Die Hydrophobisierung kann jedoch nicht in Gegenwart der spezifisch bindefähigen

4

Substanz vorgenommen werden, wenn die spezifisch bindefähige Substanz ein Protein ist und durch die Hydrophobisierung seine biologische Aktivität verliert.

Zur Hydrophobisierung durch Erhitzen werden üblicherweise Temperaturen von 40 bis 95 °C in einem Zeitraum von 1 min bis 10 Stunden angewendet, wie beispielsweise in Biochem. Biophys. Acta 624 (1980) 13-20 beschrieben.

Zur Behandlung mit Säuren sind beispielsweise Essigsäure, Propionsäure, Milchsäure oder Salzsäure geeignet. Übliche Konzentrationen sind 1 bis 100 mmol/l bei Einwirkzeiten von 10 min bis 16 Stunden.

Für die Behandlung mit chaotropen Ionen sind beispielsweise Thiocyanate, Jodide, Fluoride, Bromide, Perchlorate und Sulfate geeignet. Als denaturierende Agentien können beispielsweise Guanidinhydrochlorid oder Harnstoff verwendet werden. Üblicherweise werden hier Konzentrationen von 10 mmol/l bis 6 mol/l verwendet.

Zur Derivatisierung des Polymerisats I mit hydrophoben Verbindungen werden vorzugsweise lösliche Fettsäuren, Lipoide in nieder- oder hochmolekularer Form sowie synthetische Polymere, wie Polypropylenglykol oder lösliche Copolymere von Polystyrol eingesetzt. Die Derivatisierung erfolgt nach den dem Fachmann geläufigen Methoden.

Die Vernetzung wird mit bi- oder polyfunktionellen Verbindungen durchgeführt. Dies sind Verbindungen, die mindestens zwei funktionelle Gruppen tragen, die gleich oder verschieden sein können und die über diese funktionellen Gruppen mit funktionellen Gruppen der das Polymerisat I bildenden Verbindungen, z.B. von Proteinen reagieren können. Bevorzugt werden Verbindungen verwendet, die aus einer Alkylkette bestehen, an deren Enden sich Succinimid-, Maleinimid- und/oder Aldehydgruppen befinden.

Die Vernetzung wird dann mit der bi- oder polyfunktionellen Verbindung in an sich bekannter Weise durchgeführt.

Bevorzugt werden zur Hydrophobisierung und/oder Vernetzung Proteine mit einem Molekulargewicht von 10.000 bis 700.000 verwendet. Besonders bevorzugt wird Rinderserumalbumin, Lipase oder Immun-$\gamma$-Globulin eingesetzt.

Das auf diese Weise hergestellte Polymerisat I wird dann an ein unlösliches Trägermaterial gebunden. Die Bindung erfolgt dabei über das Polymer und ist in der Regel adsorptiv. Als Trägermaterial geeignet sind die üblicherweise verwendeten Festphasen wie Luran, Glas, Titandioxid, Polystyrol, $\gamma$-aktiviertes Polystyrol, Polystyrol-Acrylnitril-Copolymer, Papier und/oder Polyolefin. Das Trägermaterial kann vor der Weiterverarbeitung physikalisch oder chemisch vorbehandelt werden. So kann beispielsweise eine Kunststoffoberfläche vorgequollen werden oder in anderer an sich bekannter Weise aktiviert werden. Das Trägermaterial liegt in der Regel in Form von Röhrchen, Mikrotiterplatten oder Kugeln vor. Andere Ausgestaltungen sind jedoch ebenfalls möglich.

Das unlösliche Trägermaterial kann ebenso auch ein bereits beschichtetes Material sein. Geeignet sind beispielsweise mit Polystreptavidin vorbeschichtete Röhrchen oder mit polymerisiertem Antigen vorbeschichtete Röhrchen. In diesem Fall liefert das unlösliche Trägermaterial Bindungsstellen für das Polymerisat I, so daß dann die Bindung des Polymerisats I nicht nur adsorptiv erfolgt. Auf diese Weise wird eine sehr starke Bindung erhalten. Bei der adsorptiven Beschichtung ist das Polymerisat I nicht sehr stark gebunden. Dies reicht jedoch für die meisten Anwendungszwecke aus, da selbst bei Ablösung von einigen $P_1$ die Wandhaftung durch die Vernetzung mit dem Polymerisat II stabilisiert wird. Für spezielle Zwecke kann eine stärkere Bindung erwünscht sein. Für diesen Fall kann als unlösliches Trägermaterial ein vorbeschichtetes Material verwendet werden.

Das Polymerisat I wird mit einem zweiten Polymerisat II, welches eine Vielzahl von Partnern $P_2$ aufweist, vernetzt. Das Polymerisat II kann entweder nur aus $P_2$ oder aber aus einer Mischung von $P_2$ und anderen Komponenten bestehen. Das Polymerisat II weist nicht nur Bindungsstellen für $P_1$ auf, die von $P_2$ geliefert werden, sondern darüberhinaus auch Bindungsstellen für einen immunologisch nachzuweisenden Komplex, der im folgenden als Rezeptor bezeichnet werden soll. Dabei werden als Rezeptoren spezifisch bindefähige Substanzen, insbesondere entweder spezifisch bindefähige komplette Antikörper, die polyklonal oder monoklonal sein können, deren Antikörperfragmente oder Konjugate von Antikörpern oder Antikörperfragmenten mit Haptenen oder Antigenen sowie Haptene oder Antigene oder Bindeproteine (wie z.B. Thyroxin bindendes Globulin) verwendet. Die Bindungsstelle für den Rezeptor kann entweder ebenfalls von $P_2$ geliefert werden oder von einer anderen Komponente des Polymerisats II.

Die einzelnen Partner $P_2$ können über homo- oder heterobi-oder -polyvalente Verbindungen (Linker) miteinander verbunden werden. Bevorzugt wird dann die Vernetzung mit bivalenten Linkern durchgeführt, da dies eine leichtere Steuerung des Polymerisationsgrades ermöglicht. Ebenso geeignet sind jedoch auch polyvalente Linker. Als Linker können solche Verbindungen verwendet werden, die reaktionsfähige Gruppen aufweisen, die in wäßriger Lösung mit den funktionellen Gruppen der spezifisch bindefähigen Partner unter Ausbildung einer kovalenten Bindung zu reagieren vermögen. Dem Fachmann ist eine große Anzahl hierfür

EP 0 331 127 B1

geeigneter bifunktioneller oder polyfunktioneller Linker bekannt. Typische Beispiele für im Rahmen der Erfindung gut geeignete homo- oder heterobifunktionelle und -trifunktionelle Linker sind in der nachstehenden Tabelle 1 aufgeführt.

Tabelle 1

| Kurzbezeichnung | Chemische Bezeichnung |
| --- | --- |
| SPDP | N-succinimidyl 3-(2-pyridyldithio)-propionat |
| EADB | Ethyl 4-azidohenyl-1,4-dithiobutyrimidat • HCl |
| FNPA | 4-Fluoro-3-nitrophenylazid |
| HSAB | N-Hydroxysuccinimidyl-4-azidobenzoat |
| MABI | Methyl-4-azidobenzoimidat • HCl |
| MBS | m-Maleimidobenzoyl-N-Hydroxysuccinimid-ester |
| NHS-ASA | N-Hydroxysuccinimidyl-4-azidosalicylsäure |
| MHS | Maleimidohexanoyl-N-Hydroxysuccinimidester |
| PNP-DTP | p-Nitrophenyl-2-diazo-3,3,3-trifluoropropionat |
| SADP | N-Succinimidyl(4-azidophenyl)1,3'-dithiopropionat |
| SAND | Sulfosuccinimidyl-2-(m-azido-o-nitrobenzamido)ethyl-1,3'-dithiopropionat |
| SANPAH | N-succinimidyl-6(4'-azido-2'-nitrophenyl-amino)hexanoat |
| SASD | Sulfosuccinimidyl 2-(p-azidosalicylamido)ethyl-1,3'-dithiopropionat |
| SIAB | N-Succinimidyl(4-iodoacetyl)aminobenzoat |
| SMCC | Succinimidyl-4-(N-maleinimidoethyl)cyclohexan-1-carboxylat |
| SMPB | Succinimidyl-4-(p-maleimidophenyl)butyrat |
| DSS | Disuccinimidylsuberat |
| DMS | Dimethylsuberimidat |
| Traut's Reagenz | 2-Iminothiolan 2,4,6 Trichlor-s-triazin |
| SAMBA | S'-Acetyl-mercaptobernsteinsäureanhydrid |

Zur Durchführung der Vernetzung kann eine Lösung der Partner $P_2$ mit den Linkermolekülen versetzt werden unter Bedingungen, die direkt zur Vernetzung führen. Das Ausmaß der Vernetzung wird in diesem Falle durch die Menge an zugegebenem Linker gesteuert.

In einer weiteren bevorzugten Ausführungsform wird der Bindungspartner $P_2$ mit geeigneten bindefähigen Komponenten, die in bezug auf $P_1$ und den zu bestimmenden Komplex inert sind, vernetzt. Hierzu geeignet ist beispielsweise ein lösliches Protein, wie es oben definiert wurde, insbesondere Rinderserumalbumin oder Humanserumalbumin.

Die heterogene Vernetzung kann beispielsweise so erfolgen, daß sowohl das als "inerte Komponente" verwendete Proteinmaterial als auch der spezifisch bindefähige Partner $P_2$ mit einer aktivierten bindefähigen Gruppe versehen werden und anschließend umgesetzt werden. Auf diese Weise erhält man ein vernetztes Polymerisat, das ausreichend viele bindefähige Partner $P_2$ enthält. Selbstverständlich muß dabei die Bindung des Partners $P_2$ an das Protein so erfolgen, daß dadurch weder die spezifische Bindefähigkeit mit dem Partner $P_1$ beeinträchtigt wird, noch die spezifische Bindungsstelle für den zu bestimmenden Komplex blockiert wird.

In einer weiteren bevorzugten Ausführungsform des erfindungsgemäßen Verfahrens wird der Bindungspartner $P_2$ mit anderen Komponenten vernetzt, die spezifische Bindungsstellen für den Rezeptor aufweisen. Diese Ausführungsform wird bevorzugt dann verwendet, wenn $P_2$ nur eine spezifische Bindungsstelle für die Bindung mit $P_1$ aufweist. Die andere Komponente liefert dann die Bindungsstelle für den zu bindenden Rezeptor. Als andere Komponente sind Substanzen geeignet, die spezifische Bindungsstellen aufweisen, insbesondere Partner eines spezifisch bindenden Paares, wie sie oben definiert wurden.

Für die Beschichtung des unlöslichen Trägermaterials mit den Polymerisaten I und II gibt es verschiedene Varianten. In einer Ausführungsform wird das unlösliche Trägermaterial zuerst mit dem Polymerisat I beschichtet, indem das Trägermaterial mit einer Lösung, die Polymerisat I enthält, inkubiert wird. Wenn Polymerisat I gebunden ist, wird anschließend eine Lösung, die Polymerisat II enthält, zugegeben, wobei dann durch die spezifische Bindung von $P_1$ mit $P_2$ die Vernetzung der beiden Polymerisate erfolgt.

In einer anderen Ausführungsform des erfindungsgemäßen Verfahrens wird das unlösliche Trägermaterial mit einer Lösung, die sowohl Polymerisat I als auch Polymerisat II und zusätzlich einen Hemmstoff, der die Bindung von $P_1$ an $P_2$ hemmt, enthält, inkubiert. Es wird ein Hemmstoff verwendet, der reversibel wirksam ist und durch einfache Entfernung oder chemische Veränderung seine Hemmwirkung verliert. In

6

Gegenwart des Hemmstoffes können sich die Partner $P_1$ und $P_2$ völlig homogen verteilen und aufgrund der sehr langsam einsetzenden Bindung erhält man eine völlig gleichmäßige Bedeckung des Trägermaterials mit der jeweils gewünschten bindungsaktiven Substanz, die auch im großtechnischen Ansatz zu völlig gleichmäßigen und reproduzierbaren Resultaten führt. Diese Ausführungsform ist geeignet, wenn als Bindungspaar $P_1$-$P_2$ Antigen bzw. Hapten-Antikörper oder Biotin-Streptavidin bzw. Avidin eingesetzt werden. Als Hemmstoffe der Bindung von $P_1$ und $P_2$ werden im Rahmen dieser Ausführungsform der Erfindung vorzugsweise solche Substanzen verwendet, die bei chromosorptiven Aufreinigungen als Desorptionsmittel eingesetzt werden. Besonders bevorzugt werden hierzu Säuren, Basen oder chaotrope Ionen der Hofmeister-Reihe (lyotrope Reihe), wie sie beispielsweise beschrieben sind in "Structure and Stability of Biological Macromolecules", 1969, Marcel Dekker, Inc., New York, Seite 427, sowie bestimmte organische Verbindungen oder Lösungsmittel wie Acetonitril, Harnstoff oder Glycerin verwendet. Als geeignete Säuren kommen sowohl flüchtige als auch nicht flüchtige Säuren in betracht. Flüchtige Säuren können zur Aufhebung der Hemmwirkung leicht entfernt werden, z.B. durch Erwärmen, Vakuum und dergleichen. Bei nicht flüchtigen Säuren läßt sich ein analoger Effekt durch Einsatz eines Salzes einer flüchtigen Säure, welches von der nicht flüchtigen Säure zerlegt wird, unter Freisetzung der flüchtigen Säure oder durch Umpuffern erzielen. Bevorzugte Beispiele sind Essigsäure, Propionsäure und Salzsäure für flüchtige Säuren.

Ebenso lassen sich flüchtige und nicht flüchtige Basen wie z.B. Ammoniak und t-Phosphat verwenden. Weiterhin sind als Hemmstoffe organische Verbindungen geeignet, welche reversibel sowohl die Protein- als auch die Wasserstruktur beeinflussen können und beispielsweise in "J. F. Brandts, Conformatial Transitions of Proteins in Water", enthalten in "Structure and Stability of Biological Macromolecules, 1969, Marcel Dekker, Inc., New York, Seite 213-290 beschrieben sind. Besonders bevorzugt werden Glycerin und Harnstoff verwendet.

Als Hemmstoffe kommen weiterhin in Frage chaotrope Ionen, wie Thiocyanate und Jodide. Ebenfalls geeignet sind Fluoride, Bromide, Perchlorate, Guanidin und Sulfate. Ihre Entfernung zur Aufhebung der Bindungshemmung kann durch Extraktion z.B. mit organischen Lösungsmitteln oder Gemischen von organischen Lösungsmitteln oder Gemischen aus organischen Lösungsmitteln und Wasser, beispielsweise Wasser/Alkohol-Gemischen, gegebenenfalls mit Zusatz von Ionophoren und dergleichen, erfolgen. In der Regel genügt dabei bereits eine Veränderung der Ionenstärke, um den gewünschten Effekt zu erzielen, jedoch kann auch eine vollständige Entfernung des Hemmstoffes erfolgen. Auch ein Zusatz von Komplexbildnern wie EDTA kommt in Frage, z.B. zur Entfernung von hemmenden Metallsalzen wie $MgCl_2$.

Werden für Polymerisat I und II als Bindungspaar Biotin und Streptavidin bzw. Avidin eingesetzt, so wird als Hemmstoff besonders bevorzugt eine Säure eingesetzt. Die starke Bindung dieser beiden Partner kann durch Absenken des pH's auf Werte im Bereich von 4 und darunter aufgehoben werden. Bevorzugt wird dazu eine flüchtige Säure verwendet, so daß dann die Bindungswirkung bei Freisetzung der flüchtigen Säure erzielt wird.

Die beiden spezifisch bindenden Partner $P_1$ und $P_2$ und gegebenenfalls die andere Komponente, werden bevorzugt in einem solchen Verhältnis eingesetzt, daß $P_2$ und gegebenenfalls die andere Komponente für die Bindung des Rezeptors in großem Überschuß gegenüber ihren Bindungspartnern vorliegen. Auf diese Weise werden sehr viele Bindungsstellen für den zu immobilisierenden Komplex geschaffen. Wenn ein Partner des verwendeten spezifisch bindenden Paares in freier Form in der zu analysierenden Probeflüssigkeit bereits nativ vorhanden ist, ist es besonders bevorzugt, für diesen eine hohe Bindungskapazität zur Verfügung zu stellen, um dadurch eine Störung auszuschließen. Wenn also z.B. als spezifisch bindendes Paar Biotin und Streptavidin bzw. Avidin eingesetzt werden, so ist es besonders bevorzugt, eine sehr hohe Bindungskapazität für das Biotin zur Verfügung zu stellen. Biotin ist in Korperflüssigkeiten vorhanden und kann, insbesondere wenn z.B. nach Einnahme von Biotin die Serumwerte stark erhöht sind, Analysenwerte von Bestimmungen, bei denen Biotin-Konjugate eingesetzt werden, verfälschen. Für diesen Fall ist es besonders bevorzugt, eine Festphasenmatrix mit einer sehr hohen Bindungskapazität für Biotin herzustellen. Dies gelingt mit dem erfindungsgemäßen Verfahren, wobei es möglich ist, eine Bindungskapazität von 200 ng/ml und mehr für Biotin zu schaffen.

Die erfindungsgemäß hergestellte Festphasenmatrix wird bei Bestimmungen nach dem Prinzip des Immunoassays eingesetzt. Sie ist sowohl für die Varianten des Sandwich-Tests als auch für die Varianten des kompetitiven Tests geeignet. Für diese Bestimmungen gibt es viele Varianten. Dabei kann beispielsweise die Probe, die die zu bestimmende Substanz enthält mit einem Rezeptor, der eine Markierung trägt und mindestens einen weiteren Rezeptor, an den eine mit dem Polymerisat des spezifisch bindenden Partners $P_2$ spezifisch bindefähige Substanz gebunden ist, umgesetzt werden. Der zu bestimmende Komplex, vorzugsweise einer der gebundenen Rezeptoren, weist also eine Stelle auf, die mit dem Polymerisat bindefähig ist. Diese Bindungsstelle kann mit der von $P_1$ identisch sein oder aber davon verschieden. Diese Umsetzung kann bereits in einem mit der erfindungsgemäßen Matrix beschichteten Röhrchen oder einer entsprechend beschichteten Mikrotiterplatte stattfinden. Ebenso möglich ist es aber auch, die Festphasenmatrix, z.B. in Form von Kugeln, erst nach der Inkubation zuzugeben. Beim Kontakt mit der Festphasenmatrix bindet dann der Komplex aus zu bestimmender Substanz, markiertem Rezeptor und mit spezifisch bindender Substanz konjugiertem Rezeptor an das Polymerisat, das wiederum über $P_1$ und das Protein an den Träger gebunden ist. Auf diese Weise kann der zu bestimmende Komplex immobilisiert werden.

Ein weiterer Gegenstand der Erfindung ist eine Festphasenmatrix, welche dadurch gekennzeichnet ist, daß sie aus einem unlöslichen Trägermaterial besteht, an dem ein erstes Polymerisat I, das aus einer Vielzahl von Partnern $P_1$ eines spezifischen Bindungspaares und einem wasserlöslichen biologischen Polymer oder Derivat davon mit einem Molekulargewicht von mehr als etwa 20 000 besteht, gebunden ist, das mit einem zweiten Polymerisat II, das nur aus einer Vielzahl des anderen Partners $P_2$ des spezifischen Bindungspaares oder aus $P_2$, die mit anderen Komponenten vernetzt sind, besteht, wobei das Polymerisat II sowohl Bindungsstellen für $P_1$ als auch für einen immunologisch nachzuweisenden Komplex aufweist und wobei die Polymerisate I und II über die spezifische Bindung von $P_1$ mit $P_2$ vernetzt sind.

Besonders geeignet zur Durchführung von Immunoassays ist eine Festphasenmatrix, bei der das Protein ein Konjugat aus einem löslichen Protein mit einem Molekulargewicht von 200 000 bis 20 000 000 und einer Vielzahl von Biotin-, Avidin- oder Streptavidinmolekülen ist. Weiterhin bevorzugt wird eine Festphasenmatrix eingesetzt, bei der das Polymerisat II aus Biotin-, Avidin- oder Streptavidinmolekülen besteht. Das Polymerisat II wird bevorzugt gebildet aus Biotin-, Avidin- oder Streptavidinmolekülen einerseits und einem hydrophobisierten Protein andererseits.

Erfindungsgemäß wird eine Universalmatrix zur Verfügung gestellt sowie ein Verfahren zu ihrer Herstellung, die in allen bekannten Immunoassays verwendet werden kann.

Diese Festphasenmatrix ist unabhängig von der Art der verwendeten Rezeptoren. Außerdem zeichnet sie sich durch hohe Stabilität aus.

Die Erfindung wird noch durch Figuren und Beispiele erläutert.

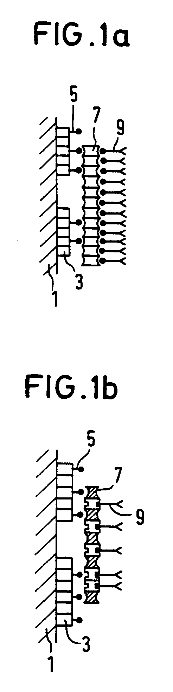

Fig 1 zeigt zwei Ausführungsformen der erfindungsgemäßen Festphasenmatrix.

a) zeigt eine Ausführungsform der erfindungsgemäßen Festphasenmatrix. An eine Festphase 1 ist ein Konjugat aus einem Protein 3 mit einem Partner eines spezifischen Bindungspaares $P_1$ 5 adsorbiert. An 5 ist ein Polymerisat 7 gebunden, das aus dem anderen Partner $P_2$ des spezifischen Bindungspaares besteht. Das Polymerisat besteht aus homogen vernetzten identischen Molekülen. An dieses Polymerisat binden bei Durchführung eines Immunoassays Antikörper 9, die mit $P_1$ konjugiert sind.

b) zeigt eine weitere Ausführungsform der erfindungsgemäßen Festphasenmatrix. Hier ist an eine Festphase 1 ein Konjugat aus einem Protein 3 und einem Partner eines spezifischen Bindungspaares $P_1$ 5 adsorbiert. An 5 ist ein Polymerisat 7 gebunden. Dieses Polymerisat besteht aus dem anderen Partner des spezifischen Bindungspaares $P_2$ sowie einem Rezeptor R. An dieses Polymerisat binden bei Durchführung eines Immunoassays Antikörper 9, die mit einer mit dem Rezeptor R bindefähigen Substanz konjugiert sind.

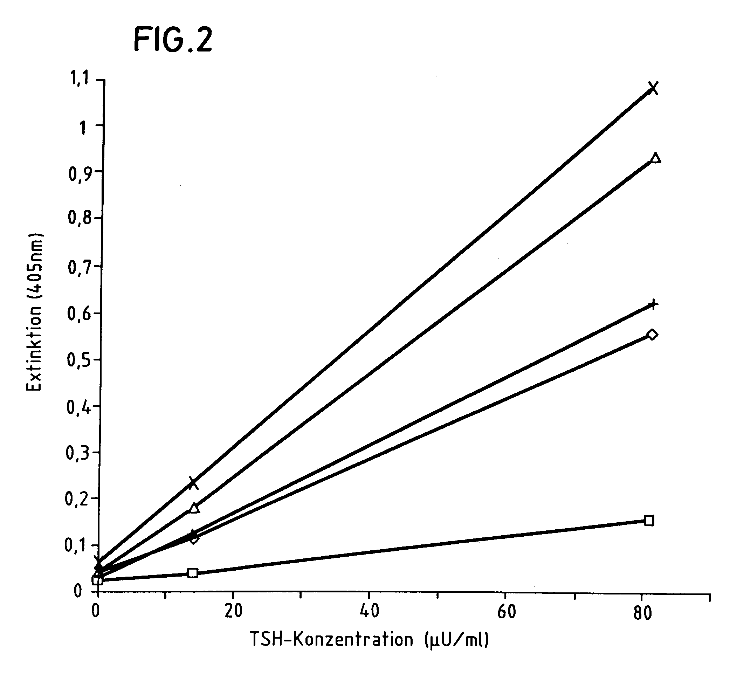

Fig 2 zeigt ein Diagramm, in dem Eichkurven für verschieden beschichtete Röhrchen aufgestellt wurden.

Die einzelnen Kurven wurden dabei mit den folgenden Röhrchen erhalten:

X: Luranröhrchen, beschichtet mit einer Zweikomponentenmatrix bestehend aus Thermo-RSA-Biotin und homogen vernetztem Streptavidin.

Dreieck: Luranröhrchen, beschichet mit einer Zweikomponentenmatrix bestehend aus Thermo-RSA-Biotin und Thermo-RSA-Streptavidin

+: Polystyrolröhrchen, gamma-bestrahlt und beschichtet mit RSA-Streptavidin.

Raute: Polystyrolröhrchen, gamma-bestrahlt und beschichtet mit homogen vernetztem Streptavidin.

Quadrat: Luranröhrchen, beschichtet mit homogen vernetztem Streptavidin.

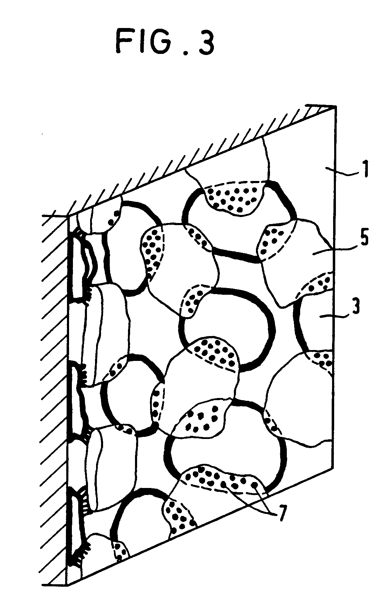

Fig. 3 zeigt eine Ausführungsform der erfindungsgemäßen Festphasenmatrix.

Bei dieser Ausführungsform ist an ein Trägermaterial 1 eine erste Komponente 3, die eine Vielzahl von Partnern eines spezifischen Bindungspaares aufweist, adsorptiv gebunden. Eine zweite Komponente 5, die eine Vielzahl der anderen Partner des spezifischen Bindungssystems enthält, ist über die spezifischen Bindungen 7 der beiden Partner an die erste Komponente 3 gebunden, wobei auf dem Trägermaterial 1 vernetzte Polymere von beträchtlicher Größe entstanden sind.

**Beispiel 1**

1a) Herstellung von Thermo-Rinderserumalbumin-Biotin

1 g Rinderserumalbumin (RSA) wird in 50 ml 50 mM Kaliumphosphat (pH 7,8) gelöst. Unter Rühren werden 1,9 ml D-Biotinyl-$\epsilon$-aminocapronsäure-N-hydroxysuccinimidester (NHS-X-Biotin, Boehringer Mannheim GmbH) in Dimethylsulfoxid (DMSO) (20 mg/ml) zugetropft. Anschließend wird 3 Stunden bei 25°C inkubiert. Nach der Reaktion wird über Nacht bei 4°C gegen das 50fache Volumen an 20 mM Kaliumphosphat (pH 7,0) dialysiert. Das Retentat wird mit dem gleichen Volumen an 20 mM Kaliumphosphat/200 mM Natriumchlorid (pH 7,0) versetzt, auf 70°C erhitzt und 4 Stunden unter vorsichtigem Rühren bei dieser Temperatur inkubiert. Anschließend wird die Lösung auf Raumtemperatur abgekühlt und filtriert. Das Filtrat wird über Nacht bei 4°C gegen das 50fache Volumen an 2 mM Kaliumphosphat (pH 7,0) dialysiert und anschließend lyophilisiert. Das erhaltene Produkt wird an der Festphase adsorbiert und stellt in der erfindungsgemäßen Matrix den an das lösliche Protein gebundenen Partner $P_1$ dar.

1b) Aktivierung von Streptavidin mit Maleimido-hexanoyl-N-hydroxysuccinimidester

30 mg Streptavidin werden in 3 ml 30 mM Kaliumphosphat/100 mM Natriumchlorid (pH 7,1) gelöst und auf 25°C temperiert. Unter Rühren werden 0,15 ml Maleinimido-hexanoyl-N-hydroxysuccinimidester (MHS) (Boehringer Mannheim GmbH) in DMSO (10 mg/ml) zugetropft. Nach einer Reaktionszeit von 1 Stunde bei 25°C wird die Lösung im Eisbad abgekühlt. Anschließend wird bei 4°C zweimal gegen 1 l 50 mM Kaliumphosphat/100 mM Natriumchlorid (pH 5,0) dialysiert.

1c) Aktivierung von Streptavidin mit S-Acetylmercaptobernsteinsäureanhydrid

30 mg Streptavidin werden in 3 ml 100 mM Kaliumphosphat (pH 7,8) gelöst und auf 25° temperiert. Unter Rühren werden 0,175 ml S-Acetylmercaptobernsteinsäureanhydrid (SAMBA) in DMSO (10 mg/ml) zugetropft. Nach einer Reaktionszeit von 3 Stunden bei 25°C wird bei 4°C zweimal gegen 1 l 50 mM Kaliumphosphat/2 mM EDTA (pH 6,5) dialysiert.

1d) Homogene Vernetzung von Streptavidin

3 ml einer Lösung von aktiviertem SAMBA-Streptavidin (10 mg/ml) (Herstellung nach Beispiel 1c)) werden auf 25° temperiert und mit 50 $\mu$l 1M Hydroxylamin (pH 6,5) versetzt. Nach 30 Minuten bei 25°C wird durch Zugabe von 15 ml 50 mM Kaliumphosphat/100 mM Natriumchlorid/1 mM EDTA (pH 6,5) verdünnt. Die homogene Vernetzung des Streptavidins wird durch Zugabe von 3 ml aktiviertem MHS-Streptavidin (10 mg/ml) (Herstellung nach Beispiel 1b)) gestartet. Nach einer Reaktionszeit von 2 Stunden bei 25°C unter vorsichtigem Rühren wird die Reaktion durch Zugabe von 0,2 ml 100 mM Cystein/HCl beendet. Nach einer Inkubationszeit von 30 Minuten bei 25°C wird der pH-Wert der Lösung durch Zugabe von 1 M Dikaliumhydrogenphosphat auf 7,5 eingestellt. Nach Zugabe von 0,2 ml 500 mM Jodacetamid wird eine weitere Stunde bei 25°C inkubiert. Anschließend wird bei 4°C zweimal gegen 3 l 50 mM Kaliumphosphat/100 mM Natriumchlorid (pH 7,5) dialysiert. Das Konjugat wird nach der Dialyse in einer Ultrafiltrationszelle aufkonzentriert.

Das homogen vernetzte Streptavidin kann entweder direkt oder nach Gelfiltration (Superose 6 prep, grade, Pharmacia Uppsala) und erneuter Aufkonzentrierung zur Adsorption an die Festphase eingesetzt werden. In der erfindungsgemäßen Matrix stellt es Polymerisat II dar.

**Beipiel 2**

Es wird heterogen vernetztes Streptavidin hergestellt, das als Polymerisat II in der erfindungsgemäßen Matrix zur Verwendung gelangt.

a) Herstellung von aktiviertem SAMBA-Thermo-RSA

Die Herstellung von Thermo-RSA erfolgt, wie unter Beispiel 1 a) beschrieben, wobei hier aber die Biotinylierung entfällt.

68 mg Thermo-RSA werden in 2 ml 0,1 M Kaliumphosphat (pH 7,8) gelöst und langsam mit 0,38 ml SAMBA (10 mg/ml in DMSO) versetzt. Nach einer Reaktionszeit von 3,5 Stunden bei 25°C wird bei 4°C zweimal gegen 1 l 50 mM Kaliumphosphat (pH 6,5) dialysiert.

b) Herstellung des Thermo-RSA-Streptavidin-Konjugats

Die heterogene Vernetzung von Streptavidin mit Thermo-RSA erfolgt analog der unter dem Beispiel 1d) beschriebenen homogenen Vernetzung. Dabei werden 60 mg aktiviertes MHS-Streptavidin (Herstellung nach Beispiel 1b)) mit 68 mg aktiviertem SAMBA-Thermo-RSA (s.o.) umgesetzt. Das Reaktionsprodukt wird durch Gelfiltration (Superose 6 prep, grade) aufgereinigt und in einer Ultrafiltrationszelle aufkonzentriert. Das erhaltene Produkt wird anschließend lyophilisiert. Das Produkt kann als Polymerisat II eingesetzt werden.

**Beispiel 3**

Beladung von Luran®- (Polystyrol-Acrylnitril-Copolymer) bzw. von γ-bestrahlten Polystyrol-Röhrchen

Die gemäß Beispiel 1 und Beispiel 2 erhaltenen Produkte werden in 50 mM Kaliumphosphat (pH 7,4) zu einer Konzentration von 10 μg/ml gelöst. Dann wird in jedes zu beladende Röhrchen 1,5 ml einer Lösung des gemäß Beispiel 1a) hergestellten Thermo-RSA-Biotin-Konjugats gefüllt und zunächst für 3 bis 5 Stunden beladen. Anschließend werden nach vollständigem Absaugen in die Röhrchen 1,5 ml einer Lösung von homogen vernetztem Streptavidin gemäß Beispiel 1 bzw. heterogen vernetztem Streptavidin gemäß Beispiel 2 gegeben und über Nacht bei Raumtemperatur inkubiert. Danach werden die Röhrchen vollständig entleert und für die entsprechenden Tests eingesetzt.

Zum Vergleich werden Röhrchen nur mit gemäß Beispiel 1 vernetztem Streptavidin oder einem Konjugat aus (nicht thermisch aggregiertem) RSA und Streptavidin (Herstellung analog dem Beispiel 2 aus aktiviertem SAMBA-RSA und aktiviertem MHS-Streptavidin) beladen, ohne Vorbeladung mit biotinyliertem Polymerisat I.

**Beispiel 4**

Es wird die Bindekapazität der gemäß Beispiel 3 hergestellten Röhrchen bestimmt.

Die mit den unterschiedlichen Streptavidinpolymerisaten erfindungsgemäß beladenen Röhrchen sowie die Vergleichsröhrchen werden mit 1 ml einer Lösung von biotinylierter Peroxidase aus Meerrettich (Biotin-POD, Sigma) (10 mU/ml in 50 mM Kaliumphosphat/0,5% Rinderserumalbumin (pH 7,4)) für 45 Minuten bei Raumtemperatur inkubiert. Die Röhrchen werden dann entleert und zweimal mit bidestilliertem Wasser gewaschen. Anschließend erfolgt die Nachweisreaktion mit Hilfe von ABTS® (Ammoniumsalz der 2,2'-azino-di(3-ethylbenzothiazolin-6-sulfonsäure)) für 30 Minuten bei Raumtemperatur. Die Messung erfolgt photometrisch bei 405 nm. Die Bindekapazität (Bika) wird über eine Verdrängungskurve ermittelt. Dazu werden der Biotin-POD-Lösung steigende Konzentrationen (0 bis 15 bzw. 0 bis 200 ng/ml) an D-Biotin (Sigma) zugesetzt. Die Bindekapazität wird dann aus der durch Auftragen der einzelnen Werte erhaltenen Kurve aus der halbmaximalen Extinktion errechnet.

**Beispiel 5**

Die Stabilität der Oberflächenhaftung der mit Protein und Biotin und jeweils einem Streptavidin-Polymerisat beschichteten Matrix wird durch Inkubation der beladenen Röhrchen mit 1,5 ml eines Detergenz enthaltenden Ablösepuffers (0,2% Tween® 20 in 50 mM Kaliumphosphat (pH 7,0)) geprüft. Nach einer Inkubationszeit von einer Stunde bei Raumtemperatur wird zur Bestimmung der abgelösten Konjugatmenge jeweils 1 ml aus den Röhrchen in ein mit Thermo-RSA-Biotin (Herstellung nach Beispiel 1a)) beschichtetes Röhrchen überführt. Parallel werden zur Ermittlung einer Eichkurve Thermo-RSA-Biotin-Röhrchen mit 1 ml Ablösepuffer versetzt, der steigende Konzentrationen an Streptavidin enthält. Nach einer Inkubationszeit von einer Stunde bei Raumtemperatur werden die Röhrchen vollständig entleert und mit 1 ml einer Biotin-POD-Lösung (100 mU/ml in 50 mM Kaliumphosphat (pH 7,0) versetzt. Nach einer weiteren Inkubation von 30 Minuten bei Raumtemperatur werden die Röhrchen entleert und anschließend dreimal mit bidestilliertem Wasser gewaschen. Die Menge an gebundener Biotin-POD ist proportional der von der Röhrchenwand abgelösten Konjugatmenge und wird durch die Substratreaktion mit ABTS (Inkubation eine Stunde bei Raumtemperatur) photometrisch bestimmt. Anhand der Eichkurve wird die Menge des abgelösten Konjugats quantifiziert und als abgelöste Biotin-Bindekapazität bezeichnet.

Tabelle 2 zeigt für verschieden beladene Luran bzw. γ-bestrahlte Polystyrol-Röhrchen (PS) die nach Beispiel 4 bestimmten Biotin-Bindekapazitäten und die nach Beispiel 5 bestimmten Desorptionen der Konjugate. Die Biotin-Bindekapazität (und damit die Bindekapazität für biotinylierte Antikörper) des direkt auf die Festphase gebundenen homogen vernetzten spezifischen Bindepartners (Vergleich) (im Beispiel Poly-Streptavidin) ist deutlich größer als die des direkt auf die Festphase gebundenen heterogen vernetzten spezifischen Bindepartners (Vergleich) (im Beispiel RSA-Streptavidin, Herstellung analog dem Beispiel 2) aus aktiviertem SAMBA-RSA und aktiviertem MHS-Streptavidin). Wie die Ablösungsdaten zeigen, kann der vernetzte spezifische Bindepartner $P_2$ jedoch nur dann mit hoher Bindekapazität und gewünscht fester Wandhaftung aufgebracht werden, wenn vorher eine Beladung mit vorvernetztem Protein, das den spezifischen Bindepartner $P_1$, d.h. Biotin kovalent gebunden enthält, in situ umgesetzt wird. Der Einfluß der Bindekapazitäten und Festphasen-Ablösungen der verschiedenen Konjugate auf die Empfindlichkeit eines unter Verwendung von Detergentien durchgeführten Funktionstests wird in Beispiel 6 näher beschrieben.

## T a b e l l e   2

| Beschich-tung | RSA-SA (Ver-gleich) | pSA (Ver-gleich) | pSA (Ver-gleich) | T-RSA-Biotin[1] + T-RSA-SA[2] (Erfindung) | T-RSA-Biotin[1] + pSA[2] (Erfindung) |
|---|---|---|---|---|---|
| Röhrchen-Material | PS | PS | Luran | Luran | Luran |
| Belade-Kon-zentration ($\mu$g/ml) | 10 | 10 | 10 | $10^1/8^2$ | $10^1/8^2$ |
| Biotin-Bika (ng) | 7,5 | 58 | 60 | 15 | 120 |
| Desorption (Bi-Bika) (ng) | 0,017 | 0,267 | 9,3 | 0,005 | 0,015 |
| % Bi-Bika-Verlust | 0,3 | 0,5 | 15,5 | 0,03 | 0,01 |

Abkürzungen: RSA–SA = Rinderserumalbumin–Streptavidin–Konjugat

pSA = polymeres, homogen vernetztes Streptavidin

T–RSA–Biotin bzw. –SA = Thermo–RSA–Biotin bzw.

–Streptavidin

$\int$–PS = $\int$-bestrahlte Polystyrolröhrchen

Biotin–Bika bzw. Bi–Bika = Biotin–Bindekapazität

## Beispiel 6

Die gemäß Beispiel 3 erhaltenen Röhrchen werden in einem TSH-Test eingesetzt.

Reagentien:

Reagenz 1 (Antikörper-Inkubationslösung)

16 mmol/l Phosphatpuffer pH 6,9

1,5 $\mu$g/ml biotinylierter monoklonaler Antikörper gegen TSH (ECACC 87122201)(Die Biotinylierung erfolgte gemäß JACS 100 (1978), 3585-3590 mit Biotin durch Umsetzung mit N-Hydroxysuccinimidbiotin im Verhältnis 10 : 1.)

Reagenz 2 (Antikörper-POD-Konjugatlösung)

36 mmol/l Phosphatpuffer pH 6,9

2,0 U/ml Konjugat aus POD und monoklonalen Antikörpern gegen TSH (ECACC 87122202)

Reagenz 3 (Substrat-Chromogen-Lösung)

100 mmol/l Phosphat-Citrat-Puffer pH 4,4

3,2 mmol/l Natriumperborat

1,9 mmol/l ABTS® (2,2'-Azino-di-[3-ethyl-benzthiazolin-sulfonsäure(6)]-diammoniumsalz)

Als Festphase werden Röhrchen, welche wie in Beispiel 3 beschrieben mit verschiedenen Matrices beschichtet worden sind, verwendet. In diese Röhrchen werden 0,2 ml Probe (TSH-Standard), 0,9 ml Reagenz 1 und 0,1 ml Reagenz 2 zugegeben und zwei Stunden bei Raumtemperatur inkubiert. Anschließend werden die Röhrchen vollständig entleert und dreimal mit Wasser gewaschen. Die an der Röhrchenwand gebundene POD-Aktivität wird dann nach Zugabe von 1 ml Reagenz 3 und einstündiger Inkubation durch Messung der Extinktion bei 405 nm ermittelt. Die Intensität der Farbreaktion ist proportional der TSH-Konzentration des Standards. Die Ergebnisse sind in Figur 2 dargestellt.

Wie Fig. 2 zeigt, nimmt die Steigung der Eichkurve (und damit die Empfindlichkeit des Tests) bei Verwendung detergenzhaltiger Inkubationspuffer von Röhrchen, die mit einer Einkomponenten-Matrix beladen sind, zu Röhrchen, die mit der erfindungsgemäßen Zweikomponenten-Matrix beladen sind, deutlich zu. Weiterhin wird die größte Empfindlichkeit durch Verwendung der Zweikomponenten-Matrix erreicht, die als Komponente B einen homogen vernetzten Bindepartner (hier polymeres Streptavidin) enthält.

## Beispiel 7

Es wird eine Matrix hergestellt, bei der auf die Festphase Thermo-RSA adsorbiert wird, an das Streptavidin als Partner $P_1$ gebunden ist. An das Streptavidin wird dann homogen vernetztes, biotinyliertes Protein A als Partner $P_2$ gekuppelt.

a) Herstellung von Thermo-RSA-Streptavidin

Die Herstellung erfolgt, wie unter Beispiel 2 beschrieben.

b) Herstellung von homogen vernetztem, biotinyliertem Protein A

50 mg Protein A (Boehringer Mannheim GmbH) werden in 5 ml 30 mM Kaliumphosphat (pH 7,1) gelöst und mit dem 10fachen molaren Überschuß an NHS-X-Biotin (gelöst zu 10 mg/ml in DMSO) versetzt. Nach einer Inkubationszeit von einer Stunde bei 25°C wird das Reaktionsgemisch bei 4°C über Nacht gegen 10 l 50 mM Kaliumphosphat (pH 8,0) dialysiert. Das Retentat wird anschließend in einer Ultrafiltrationszelle auf eine Konzentration von 50 mg Biotin-Protein A/ml konzentriert.

Die konzentrierte Lösung von Biotin-Protein A wird auf 25°C erwärmt. Anschließend werden unter vorsichtigem Rühren 50 $\mu$l einer Disuccinimidylsuberat-Lösung (DSS, Fa. Pierce; 7 mg/ml in Dioxan) zugegeben. Die Vernetzung wird durch HPLC an einer TSK 3000 Gelfiltrationssäule (LKB) kontrolliert. In Abständen von einer Stunde werden so lange jeweils 50 $\mu$l der DSS-Lösung zugegeben, bis der Peak des monomeren Protein A auf weniger als 10% seiner Ausgangsgröße reduziert worden ist. Danach wird die weitere Vernetzung durch Zugabe von 50 $\mu$l 1M Ethanolamin (pH 8,0) gestoppt. Es wird über Nacht bei 4°C inkubiert und anschließend zweimal gegen 2 l 2 mM Kaliumphosphat (pH 7,5) dialysiert. Die Abtrennung des monomeren Protein A erfolgt durch Gelfiltration an Superose 12 prep. grade. Das homogen vernetzte Produkt wird gesammelt und in einer Ultrafiltrationszelle konzentriert. Mit diesen beiden Komponenten werden dann Röhrchen gemäß Beispiel 3 beladen.

**Beipiel 8**

Es wird eine Matrix, bestehend aus einem Konjugat von Thermo-RSA mit Fc$\gamma$-Fragmenten der Maus als P$_1$ und einem homogen vernetzten polyklonalen Anti-Maus-Fc$\gamma$-Antikörper aus Schafen als P$_2$ hergestellt.

a) Herstellung von Thermo-RSA-Maus-Fc$\gamma$-Fragment

Die Fc$\gamma$-Fragmente werden durch Papain-Spaltung von Immunglobulinen G der Maus und Abtrennung von den Fab-Fragmenten durch Ionenaustausch-Chromatographie an DE-52-Cellulose nach üblichem Verfahren präpariert.

Aktiviertes MHS-Fc$\gamma$-Fragment wird analog der Herstellung von aktiviertem MHS-Streptavidin (Beispiel 1b)) hergestellt. Aktiviertes SAMBA-Thermo-RSA wird, wie im Beispiel 2 beschrieben, präpariert. Die Konjugation von 68 mg aktiviertem SAMBA-Thermo-RSA mit 10 mg aktiviertem MHS-Fc$\gamma$-Fragment erfolgt auf gleiche Weise wie die Herstellung von heterogen vernetztem Streptavidin (Beispiel 2). Das Reaktionsprodukt wird durch Gelfiltration an Superose 6 prep grade aufgereinigt, in einer Ultrafiltrationszelle konzentriert und anschließend lyophilisiert.

b) Herstellung von homogen vernetztem Anti-Maus-Fc$\gamma$-Antikörper

50 mg Anti-Maus-Fc$\gamma$-Antikörper werden in 1 ml 50 mM Kaliumphosphat pH 8,0 gelöst und auf 25°C erwärmt. Analog der Herstellung von homogen vernetztem Protein A (Beispiel 7b) werden in Abständen von einer Stunde jeweils so lange 50 $\mu$l einer DSS-Lösung (7 mg/ml in Dioxan) zugesetzt, bis in der HPLC-Analytik an einer TSK 3000 Gelfiltrationssäule der Peak des monomeren IgGs auf 10% seiner Ausgangsgröße gesunken ist. Anschließend wird, wie unter Beispiel 7b) beschrieben, mit Ethanolamin gestoppt und dialysiert. Das monomere IgG wird, wie in Beispiel 7b) beschrieben, durch Gelfiltration abgetrennt. Gegebenenfalls wird das vernetzte IgG durch Ultrafiltration konzentriert. Alternativ kann das homogen vernetzte Produkt durch Aktivierung des Antikörpers mit MHS und SAMBA (Herstellung analog den Beispielen 1b) und 1c)) und anschließender Vernetzung (analog Beispiel 1d)) präpariert werden.

Mit diesen gemäß a) und b) erhaltenen Produkten werden Röhrchen gemäß Beispiel 3 beladen.

**Beispiel 9**

Es wird eine Matrix, bestehend aus einem Thermo-RSA-Digitoxigenin-Konjugat als Polymerisat I und homogen vernetztem Anti-Digoxin-Antikörper aus Schaf als Polymerisat II hergestellt.

a) Herstellung von Thermo-RSA-Digitoxigenin

Thermo-RSA wird, wie im Beispiel 1a) beschrieben, hergestellt, wobei hier aber die Biotinylierung entfällt. 68 mg Thermo-RSA werden in 6,8 ml 50 mM Kaliumphosphat/100 mM Natriumchlorid (pH 8,5) gelöst und auf 25°C erwärmt. Unter Rühren werden 2,86 mg Digitoxigenin-3-succinimidyl-hydroxysuccinimidester in 0,68 ml Dioxan zugegeben. Nach einer Reaktionszeit von 3 Stunden bei 25°C wird zweimal gegen 1 l 2 mM Kaliumphosphat (pH 7,2) dialysiert. Anschließend wird das Reaktionsprodukt in einer Ultrafiltrationszelle konzentriert.

b) Herstellung von homogen vernetztem Anti-Digoxin-Antikörper

Die Herstellung des homogen vernetzten Anti-Digoxin-Antikörpers erfolgt auf gleiche Weise wie für die Herstellung des homogen vernetzten Anti-Maus-Fcγ-Antikörper (Beispiel 8b)) beschrieben.
Anschließend werden Röhrchen nacheinander mit Lösungen der beiden Produkte beladen.
Im Immunoassay werden Digitoxigenin-markierte Antikörper eingesetzt. Die Herstellung derartig markierter Antikörper erfolgt analog Beispiel 9 a mit Digitoxigenin-3-succinimidyl-hydroxysuccinimidester.

**Beispiel 10**

Es wird eine Matrix hergestellt, indem die Festphase gleichzeitig mit Polymerisat I und II inkubiert wird.
Eine Lösung, bestehend aus 10 $\mu$g/ml poly-Streptavidin (Herstellung nach Beispiel 1d) und 0,1 $\mu$g/ml Thermo-RSA-Biotin (Herstellung nach Beispiel 1a) in 5 mmol/l Essigsäure wird 20 Stunden in Polystyrol-Tubes inkubiert.
Nach Entfernung der Beladelösung wird mit 10 mmol/l Kaliumphosphat-Puffer, pH 7,2 / 3 g RSA nachbeladen (30 Minuten) und nach Absaugen getrocknet.

**Beispiel 11**

a) Biotinylierung von Amino-Dextran-500

100 mg Aminodextran, (MW 500.000, 230 $NH_2$-Gruppen/Mol Dextran) werden in 5 ml 100 mM Kaliumphosphat-Puffer, pH 8,5, gelöst. Dazu werden 90 $\mu$l einer Lösung aus 10 mg Biotin-N-hydroxysuccinimidester (Biotin-OSu) in 1 ml Dimethylsulfoxid (DMSO) langsam unter Rühren zugegeben.
Nach 2 Stunden bei Raumtemperatur wird die Reaktion mit 50 $\mu$l 200 mmol/l Lysin-HCl, pH 8,5 gestoppt.
Anschließend wird der Ansatz zweimal gegen das 500fache Volumen 50 mmol/l Kaliumphosphat-Puffer, pH 7,2 dialysiert und mit 40 mmol/l Kaliumphosphat-Puffer, pH 7,0 auf eine Konzentration von 10 $\mu$g/ml verdünnt.

b) Beladung von Polystyrol-Tubes mit Amino-Dextran-Biotin

Jeweils 1,5 ml dieser Beladelösung werden in Polystyrol-Röhrchen gefüllt und bei Raumtemperatur 6 Stunden inkubiert.
Im Anschluß daran wird abgesaugt, 1,5 ml einer homogen oder heterogen vernetztem Poly-Streptavidin (Beispiel 1 oder 2) zugegeben und über Nacht inkubiert. Danach werden die Röhrchen vollständig entleert und für die entsprechenden Tests eingesetzt.

**Beispiel 12**

Thermo-RSA-Streptavidin-Tubes, hergestellt nach EP-A 0 269 092, werden mit 10 $\mu$g/ml Thermo-RSA-Biotin-Lösung (Herstellung nach Beispiel 1a) in 50 mmol/l Kaliumphosphat, pH 7,2, 20 Stunden inkubiert.
Nach vollständigem Absaugen der Lösung wird 1,5 ml Poly-Streptavidin-Lösung (nach Beispiel 1 oder 2) zugegeben und über Nacht bei Raumtemperatur inkubiert. Danach werden die Röhrchen vollständig entleert und für die entsprechenden Tests eingesetzt.

**Beispiel 13**

a) Biotinylierung von Poly(Lys/Phe)

100 mg Poly(Lys/Phe)HBr (Hydrobromid eines Copolymeren aus Lysin und Phenylalanin) (L-Lys/L-Phe 1:1, MW 46 KD, Hersteller Sigma) werden in 25 ml $H_2O$ gelöst.
Dazu werden 180 $\mu$l einer Lösung aus 5 mg Biotin-OSu in 1 ml DMSO langsam unter Rühren zugegeben, wobei der pH mittels Autotitrator bei 8,0 konstant gehalten wird.

15

Nach 2 Stunden bei Raumtemperatur wird die Reaktion mit 50 $\mu$l 200 mmol/l Lysin-HCl, pH 8,5, gestoppt.

Anschließend wird der Ansatz zweimal gegen das 500fache Volumen bidest. $H_2O$ dialysiert und auf eine Konzentration von 10 $\mu$g/ml verdünnt.

b) Beladung von Polystyrol-Tubes mit Poly(Phe/Lys)Biotin

Jeweils 1,5 ml dieser Beladelösung werden in $\gamma$-bestrahlten Polystyrol-Röhrchen gefüllt und bei Raumtemperatur 6 Stunden inkubiert. Im Anschluß daran wird abgesaugt und 1,5 ml Poly-Streptavidin-Lösung (Herstellung nach Beispiel 1 oder 2) zugegeben und bei Raumtemperatur über Nacht inkubiert. Danach werden die Röhrchen vollständig entleert und für die entsprechenden Tests eingesetzt.

**Beispiel 14**

Es wird eine Matrix, bestehend aus einem Thermo-RSA-Biotin-Konjugat als Polymerisat I und einem heterogen vernetztem Konjugat aus Streptavidin und Anti-Digoxin-Antikörpern aus Schaf als Polymerisat II hergestellt.

a) Herstellung von Thermo-RSA-Biotin

Die Herstellung erfolgt, wie unter Beispiel 1a) beschrieben. Das Thermo-RSA-Biotin-Konjugat stellt in der erfindungsgemäßen Matrix das Polymerisat I dar.

b) Herstellung des Mischpolymerisats aus Streptavidin und Anti-Digoxin-Antikörpern aus Schaf.

30 mg Streptavidin werden wie unter Beispiel 1c) beschrieben, mit S-Acetylmercaptobernsteinsäureanhydrid (SAMBA) aktiviert.

Gleichzeitig werden 30 mg Anti-Digoxin-Antikörper auf gleiche Weise mit Maleimido-hexanoyl-N-hydroxysuccinimidester (MHS) aktiviert, wie es in Beispiel 1b) für die MHS-Aktivierung von Streptavidin beschrieben ist. Für die Umsetzung wird ein fünffach molarer Überschuß an MHS (gelöst in DMSO) gegenüber den Antikörpern eingesetzt.

Die Konjugation von aktiviertem SAMBA-Streptavidin mit aktivierten MHS-Antikörpern erfolgt auf die gleiche Weise wie es für die homogene Vernetzung von Streptavidin in Beispiel 1d) beschrieben ist.

Das auf diese Weise erhaltene Streptavidin-Antikörper-Konjugat kann entweder direkt oder nach Gelfiltration und erneuter Aufkonzentrierung zur Adsorption an die Festphase eingesetzt werden. In der erfindungsgemäßen Matrix stellt es das Polymerisat II dar.

Zur Herstellung der erfindungsgemäßen Matrix werden Röhrchen entweder nacheinander mit Lösung der beiden Produkte, oder aber gleichzeitig, wie in Beispiel 10 beschrieben, in Anwesenheit von 5 mmol/l Essigsäure beschichtet.

Im Immunoassay werden Digitoxigenin-markierte Antikörper eingesetzt. Die Herstellung derartig markierter Antikörper erfolgt analog Beispiel 9a mit Digitoxigenin-3-succinimidyl-hydroxysuccinimidester.

**Patentansprüche**

**1.** Verfahren zur Herstellung einer an ein unlösliches Trägermaterial gebundenen, spezifisch bindefähigen Substanz, insbesondere für die Verwendung in einem heterogenen Analysenverfahren nach dem Prinzip des Immunoassays,
**dadurch gekennzeichnet,**
daß man ein erstes Polymerisat I, das aus einer Vielzahl von Partnern $P_1$ eines spezifischen Bindungspaares und einem wasserlöslichen biologischen Polymer oder Derivat davon mit einem Molekulargewicht von mehr als etwa 20 000 besteht, an ein unlösliches Trägermaterial bindet und mit einem zweiten Polymerisat II, das nur aus einer Vielzahl von Molekülen des anderen Partners $P_2$ des spezifischen Bindungspaares oder aus $P_2$ die mit anderen Komponenten vernetzt sind, besteht, über die spezifische Bindung von $P_1$ mit $P_2$ vernetzt, wobei das Polymerisat II sowohl Bindungsstellen für $P_1$ als auch für eine immunologischen nachzuweisenden Komplex aufweist.

**2.** Verfahren nach Anspruch 1,
**dadurch gekennzeichnet,** daß man für das Polymerisat I ein Protein, Peptid, Kohlenhydrat oder Nukleinsäurepolymer oder ein Copolymerisat aus Aminosäuren und Kohlenhydraten verwendet.

3. Verfahren nach Anspruch 2,
**dadurch gekennzeichnet,** daß man für das Polymerisat I ein Protein verwendet, das hydrophober ist als Polymerisat II.

4. Verfahren nach einem der Ansprüche 1 bis 3, **dadurch gekennzeichnet,** daß man als Polymerisat II ein Polymerisat verwendet, in dem spezifische Partner $P_2$ mit anderen Komponenten vernetzt sind.

5. Verfahren nach Anspruch 4, **dadurch gekennzeichnet,** daß man $P_2$ mit anderen Komponenten zu dem Polymerisat II vernetzt, die ebenfalls spezifische Bindungsstellen aufweisen.

6. Verfahren nach Anspruch 4, **dadurch gekennzeichnet,** daß der spezifische Partner $P_2$ mit einem hydrophobisierten Protein vernetzt wird.

7. Verfahren nach einem der vorhergehenden Ansprüche, **dadurch gekennzeichnet,** daß man das unlösliche Trägermaterial zuerst mit Polymerisat I beschichtet und anschließend mit Polymerisat II vernetzt.

8. Verfahren nach einem der Ansprüche 1 bis 6,
**dadurch gekennzeichnet,** daß man eine Lösung, die das Polymerisat I und das Polymerisat II sowie einen Hemmstoff für die Bindung von $P_1$ an $P_2$ enthält, mit dem unlöslichen Trägermaterial in Kontakt bringt und nach Bindung von Polymerisat I und Polymerisat II an das Trägermaterial die Bindung von $P_1$ und $P_2$ durch Entfernen des Hemmstoffes oder Aufhebung der Hemmwirkung auslöst.

9. Festphasenmatrix,
**dadurch gekennzeichnet,** daß sie aus einem unlöslichen Trägermaterial besteht, an dem ein erstes Polymerisat I, das aus einer Vielzahl von Partnern $P_1$ eines spezifischen Bindungspaares und einem wasserlöslichen biologischen Polymer oder Derivat davon mit einem Molekulargewicht von mehr als etwa 20 000 besteht, gebunden ist, das mit einem zweiten Polymerisat II vernetzt ist, das nur aus einer Vielzahl des anderen Partners $P_2$ des spezifischen Bindungspaares oder aus $P_2$, die mit anderen Komponenten vernetzt sind, besteht, wobei das Polymerisat II sowohl Bindungsstellen für $P_1$ als auch für einen immunologisch nachzuweisenden Komplex aufweist und wobei die Polymerisate I und II über die spezifische Bindung von $P_1$ mit $P_2$ vernetzt sind.

10. Festphasenmatrix nach Anspruch 9,
**dadurch gekenneziechnet,** daß man als Polymerisat I ein Protein, Peptid, Kohlenhydrat oder Nucleinsäure-Polymer oder ein Copolymerisat aus Aminosäuren und Kohlehydraten, jeweils mit einer Vielzahl von Partnern $P_1$ eines spezifischen Bindungspaares verwendet.

11. Festphasenmatrix nach Anspruch 9 oder 10,
**dadurch gekennezeichnet,** daß das Polymerisat II ein Konjugat aus einem löslichen Protein mit einem Molekulargewicht von 200 000 bis 20 000 000 und einer Vielzahl von Biotin-, Avidin- oder Streptavidinmolekülen ist.

12. Festphasenmatrix nach einem der Ansprüche 9 bis 11,
**dadurch gekennzeichnet,** daß das Polymerisat II aus Biotin-, Avidin- oder Streptavidinmolekülen besteht.

13. Festphasenmatrix nach einem der Ansprüche 9 bis 11,
**dadurch gekennezeichnet,** daß das Polymerisat II aus Biotin-, Avidin- oder Streptavidinmolekülen und einem hydrophobisierten Protein besteht.

**Claims**

1. Process for the production of a specifically bindable substance bound to an insoluble carrier material, especially for the use in a heterogeneous analysis process according to the immunoassay principle, characterised in that one binds a first polymer I, which consists of a plurality of partners $P_1$ of a specific

binding pair and a water-soluble biological polymer or derivative thereof with a molecular weight of more than about 20,000, to an insoluble carrier material and cross-links with a second polymer II, which only consists of a plurality of molecules of the other partner $P_2$ of the specific binding pair or of $P_2$ which are cross-linked with other components via the specific binding of $P_1$ with $P_2$, whereby the polymer II not only has binding positions for $P_1$ but also for an immunological complex to be detected.

2. Process according to claim 1, characterised in that, for the polymer I, one uses a protein, peptide, carbohydrate or nucleic acid polymer or a co-polymer of amino acids and carbohydrates.

3. Process according to claim 2, characterised in that, for the polymer I, one uses a protein which is more hydrophobic than polymer II.

4. Process according to one of claims 1 to 3, characterised in that, as polymer II, one uses a polymer in which specific partners $P_2$ are cross-linked with other components.

5. Process according to claim 4, characterised in that one cross-links $P_2$ with other components to give the polymer II which also has specific binding positions.

6. Process according to claim 4, characterised in that the specific partner $P_2$ is cross-linked with a hydrophobing protein.

7. Process according to one of the preceding claims, characterised in that one first coats the insoluble carrier material with polymer I and subsequently cross-links with polymer II.

8. Process according to one of claims 1 to 6, characterised in that one brings a solution, which contains the polymer I and the polymer II, as well as an inhibitor for the binding of $P_1$ to $P_2$, into contact with the insoluble carrier material and, after binding of polymer I and polymer II to the carrier material, initiates the binding of $P_1$ and $P_2$ by removal of the inhibitor or removal of the inhibiting action.

9. Solid matrix, characterised in that it consists of an insoluble carrier material to which is bound a first polymer I, which consists of a plurality of partners $P_1$ of a specific binding pair or derivative thereof with a molecular weight of more than about 20,000 which are cross-linked with a second polymer II which only consists of a plurality of the other partner $P_2$ of the specific binding pair or of $P_2$ which are cross-linked with other components, whereby the polymer II not only has binding positions for $P_1$ but also for an immunological complex to be detected and whereby the polymers I and II are cross-linked via the specific binding of $P_1$ with $P_2$.

10. Solid phase matrix according to claim 9, characterised in that, as polymer I, one uses a protein, peptide, carbohydrate or nucleic acid polymer or a co-polymer of amino acids and carbohydrates, in each case with a plurality of partners $P_1$ of a specific binding pair.

11. Solid phase matrix according to claim 9 or 10, characterised in that the polymer II is a conjugate of a soluble protein with a molecular weight of 200,000 to 20,000,000 and a plurality of biotin, avidin or streptavidin molecules.

12. Solid phase matrix according to one of claims 9 to 11, characterised in that the polymer II consists of biotin, avidin or streptavidin molecules.

13. Solid phase matrix according to one of claims 9 to 11, characterised in that the polymer II consists of biotin, avidin or streptavidin molecules and a hydrophobing protein.

**Revendications**

1. Procédé de préparation d'une substance capable de liaison spécifique, liée à un matériau support insoluble, utilisable en particulier dans un procédé d'analyse hétérogène selon le principe des déterminations immunologiques, caractérisé en ce qu'on lie à un matériau support insoluble un premier polymère I qui consiste en une multiplicité de partenaires $P_1$ d'une paire de liaison spécifique et en un polymère biologique hydrosoluble ou un dérivé de celui-ci d'un poids moléculaire supérieur à environ 20000, et on le réticule, par l'intermédiaire de la liaison spécifique de $P_1$ avec $P_2$, avec un second polymère II qui ne consiste qu'en une multiplicité de molécules de l'autre partenaire $P_2$ de la paire de liaison spécifique ou de $P_2$ qui sont réticulées avec d'autres composants, le polymère II présentant des sites de liaison pour $P_1$ et pour un complexe qui doit être mis en évidence par voie immunologique.

2. Procédé selon la revendication 1, caractérisé en ce que l'on utilise comme polymère I une protéine, un peptide, un hydrate de carbone ou un polymère d'acide nucléique, ou un copolymère d'acides aminés et d'hydrates de carbone.

3. Procédé selon la revendication 2, caractérisé en ce que l'on utilise comme polymère I une protéine qui est plus hydrophobe que le polymère II.

4. Procédé selon l'une des revendications 1 à 3, caractérisé en ce que l'on utilise comme polymère II un polymère dans lequel des partenaires spécifiques $P_2$ sont réticulés avec d'autres composants.

5. Procédé selon la revendication 4, caractérisé en ce que l'on réticule $P_2$ en le polymère II avec d'autres composants qui présentent également des sites de liaison spécifiques.

6. Procédé selon la revendication 4, caractérisé en ce que le partenaire spécifique $P_2$ est réticulé avec une protéine rendue hydrophobe.

7. Procédé selon l'une des revendications précédentes, caractérisé en ce que l'on recouvre d'abord de polymère I le matériau support insoluble puis on le réticule avec le polymère II.

8. Procédé selon l'une des revendications 1 à 6, caractérisé en ce que l'on met en contact avec le matériau support insoluble une solution qui contient le polymère I et le polymère II ainsi qu'un inhibiteur de la liaison de $P_1$ avec $P_2$, et, après la liaison du polymère I et du polymère II sur le matériau support, on coupe la liaison de $P_1$ et $P_2$ en éliminant l'inhibiteur ou en supprimant l'effet d'inhibition.

9. Matrice en phase solide, caractérisée en ce qu'elle consiste en un matériau support insoluble sur lequel est lié un premier polymère I qui consiste en une multiplicité de partenaires $P_1$ d'une paire de liaison spécifique et en un polymère biologique hydrosoluble ou un dérivé de celui-ci d'un poids moléculaire supérieur à environ 20000 qui est réticulé avec un second polymère II qui ne consiste qu'en une multiplicité d'autres partenaires $P_2$ de la paire de liaison spécifique ou en $P_2$ qui sont réticulés avec d'autres composants, le polymère II comportant des sites de liaison pour $P_1$ et pour un complexe qui doit être mis en évidence par voie immunologique et les polymères I et II étant réticulés par l'intermédiaire de la liaison spécifique de $P_1$ avec $P_2$.

10. Matrice en phase solide selon la revendication 9, caractérisée en ce que l'on utilise comme polymère I une protéine, un peptide, un hydrate de carbone ou un polymère d'acide nucléique, ou un copolymère d'acides aminés et d'hydrates de carbone, dans chaque cas avec une multiplicité de partenaires $P_1$ d'une paire de liaison spécifique.

11. Matrice en phase solide selon la revendication 9 ou 10, caractérisée en ce que le polymère II est un conjugué d'une protéine soluble d'un poids moléculaire de 200000 à 20000000 et d'une multiplicité de molécules de biotine, d'avidine ou de streptavidine.

12. Matrice en phase solide selon l'une des revendications 9 à 11, caractérisée en ce que le polymère II consiste en molécules de biotine, d'avidine ou de streptavidine.

13. Matrice en phase solide selon l'une des revendications 9 à 11, caractérisée en ce que le polymère II consiste en molécules de biotine, d'avidine ou de streptavidine et en une protéine rendue hydrophobe.

## FIG.1a

## FIG.1b

FIG.2

EP 0 331 127 B1

# FIG . 3